# EUROPÄISCHE PATENTSCHRIFT

(11) **EP 1 185 503 B1**
(45) Veröffentlichungstag und Bekanntmachung des Hinweises auf die Patenterteilung: **14.07.2004**
(21) Anmeldenummer: 01931493.9
(22) Anmeldetag: 02.03.2001
(51) Int. Cl.: C07C 243/02, C07C 241/00, C06B 25/34

(54) **VERFAHREN ZUR HERSTELLUNG VON DINITRODIAZAALKANEN UND ZWISCHENPRODUKTEN HIERZU**
METHOD FOR PRODUCING DINITRO-DIAZA-ALKANES AND INTERMEDIATE PRODUCTS HERETO
PROCEDE DE PREPARATION DE DINITRODIAZA-ALCANES ET PRODUITS INTERMEDIAIRES APPROPRIES

(30) Priorität: 02.03.2000 DE 10010190
(43) Veröffentlichungstag der Anmeldung: 13.03.2002
(73) Patentinhaber: Nitrochemie Aschau GmbH, 84544 Aschau a.Inn (DE)
(72) Erfinder: KNOTT, Thomas, 84478 Waldkraiburg (DE)
(74) Vertreter: Lieck, Hans-Peter, Dipl.-Ing.
(86) Internationale Anmeldenummer: PCT/EP2001/002383
(87) Internationale Veröffentlichungsnummer: WO 2001/064627

(56) Entgegenhaltungen:
- US-A- 4 476 322
- DATABASE BEILSTEIN [Online] BEILSTEIN INSTITUTE FOR ORGANIC CHEMISTRY, FRANKFURT/MAIN, DE; Database accession no. 1799867 XP002172589 & CAN. J. RES. SECT. B., Bd. 25, - 1947 Seite 299
- DATABASE BEILSTEIN [Online] BEILSTEIN INSTITUTE FOR ORGANIC CHEMISTRY, FRANKFURT/MAIN, DE; Database accession no. 1803082 XP002172590 & RECL. TRAV. CHIM. PAYS-BAS, Bd. 16, - 1897 Seite 388
- DATABASE BEILSTEIN [Online] BEILSTEIN INSTITUTE FOR ORGANIC CHEMISTRY, FRANKFURT/MAIN, DE; Database accession no. 1805355 XP002172591 & RECL. TRAV. CHIM. PAYS-BAS, Bd. 17, - 1898 Seite 272
- DATABASE BEILSTEIN [Online] BEILSTEIN INSTITUTE FOR ORGANIC CHEMISTRY, FRANKFURT/MAIN, DE; Database accession no. 1801774 XP002172592 & RECL. TRAV. CHIM. PAYS-BAS, Bd. 4, - 1885 Seite 200

## Beschreibung

Seit einigen Jahren sind Treibladungspulver bekannt, die als energetischen Weichmacher, auch Sprengöl genannt, Dinitrodiazaalkane enthalten, und zwar hier insbesondere 2,4-Dinitro-2,4-Diazapentan, allein oder im Gemisch mit anderen entsprechenden Alkanen (US-PS 4,476,322, US-PS 4,457,791).

Es liegt in der Natur der Dinitrodiazaalkane, daß die damit hergestellten Treibladungspulver ein nahezu temperaturunabhängiges Abbrandverhalten haben. Dies ist eine sehr angestrebte Eigenschaft. Sie bedeutet, daß der beim Abbrand der Treibladung im System entwickelte maximale Gasdruck nur in einem geringen oder sehr geringen Maße von der Umgebungstemperatur abhängt. Treibladungspulver mit einem temperaturunabhängigen Abbrand ermöglichen die Ausnutzung des maximalen Leistungspotentials des Systems in einem entsprechend weiten Temperaturbereich.

Einer umfangreichen Verwendung von Dinitrodiazaalkanen für die Herstellung von Treibladungspulvern mit einem entsprechend ausgeglichenen Temperaturverhalten steht bisher entgegen, daß die Dinitrodiazaalkane schwierig herzustellen und dementsprechend teuer sind.

Bei einem bekannten Herstellungsverfahren (US-PS 4,476,322, dort mit weiteren Zitaten) wird 2,4-Dinitro-2,4-Diazapentan aus Dimethyl- oder Diethylharnstoff hergestellt. Der Harnstoff wird mit Salpetersäure nitriert und das Nitrierungsprodukt zu Methyl- bzw. Ethylnitramin hydrolisiert. Die erhaltenen Nitramine werden mit Hilfe von Para-Formaldehyd und Schwefelsäure zum 2,4-Dinitro-2,4-Diazapentan kondensiert. In analoger Weise können auch 2,4-Dinitro-2,4-Diazahexan und 3,5-Dinitro-3,5-Diazaheptan sowie Mischungen der drei genannten Alkane hergestellt werden (Tartakofsky et al, Russian Chemical Bulletin, 1993, 42, 1916 ff). Die Herstellung aus Harnstoff ergibt nur eine relativ geringe Gesamtausbeute und der eingesetzte Diethylharnstoff ist sehr teuer. Außerdem stellt die nitrierte Harnstoffverbindung eine äußerst instabile temperatur- und säureempfindliche, explosive Zwischenstufe dar.

Bei einem anderen vorgeschlagenen Verfahren zur Herstellung des vorgenannten Gemisches aus drei Dinitrodiazaalkanen setzt man Methyl- oder Ethylamin mit einem Chlorameisensäure-Ester unter Verwendung von Natronlauge zu einem Zwischenprodukt um, das anschließend mit Salpetersäure nitriert wird. Das Nitrierungsprodukt wird mittels Ammoniak und Ethanol im Rückfluß in Methyl- oder Ethylnitramin umgesetzt, das dann wie bei dem vorherigen Verfahren zu den Dinitrodiazaalkanen kondensiert wird. Bei diesem Verfahren ist der vorletzte Schritt der Erzeugung der Nitramine sehr aufwendig und zeitintensiv, so daß er in einem größeren technischen Maßstab nicht zu realisieren ist.

Der Erfindung liegt die Aufgabe zugrunde, ein im technischen Maßstab leicht, preiswert und ohne große Sicherheitsrisiken durchführbares Verfahren zur Herstellung von Dinitrodiazaalkanen anzugeben.

Diese Aufgabe ist erfindungsgemäß mit dem in Anspruch 1 und bezüglich vorteilhafter Ausgestaltungen in den rückbezogenen Unteransprüchen gekennzeichneten Verfahren gelöst.

Bei dem erfindungsgemäßen Verfahren wird von einem Diester, vorzugsweise einem Dialkylester einer Dicarbonsäure, vorzugsweise von Oxalsäure-Diethylester ausgegangen. Diesen setzt man mit einem primären aliphatischen Amin, vorzugsweise Ethylamin in das korrespondierende Dialkyldiamid um. Die Reaktion findet dabei im wassrigen Milieu statt. Die Reaktionstemperatur liegt zwischen 0 und 80°C. Die Dialkyldiamide fallen als Niederschlag aus und können abfiltriert werden. Die folgende Formel gibt den ersten Schritt des erfindungsgemäßen Verfahrens wieder:

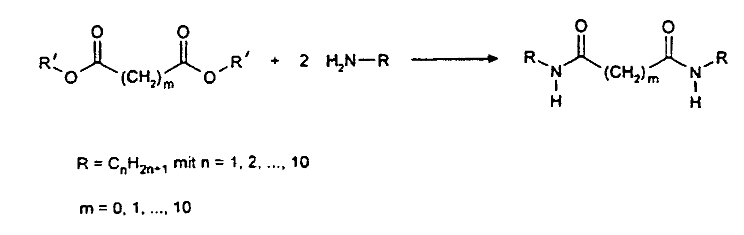

Hierbei zeigt die Formel den Fall, daß mit einem primären aliphatischen Amin ein Dialkyldiamid der Dicarbonsäure gebildet wird. Statt Diamiden mit aliphatischen Resten können jedoch auch Diamide der Dicarbonsäure mit zyklischen oder aromatischen Resten gebildet werden, was durch Wahl eines entsprechenden zyklischen oder aromatischen Amins gesteuert wird.

Im zweiten Schritt des erfindungsgemäßen Verfahrens werden die erhaltenen Dialkyldiamide mittels üblicher Nitrierungsmittel zu korrespondierenden Dialkyldinitroamiden nitriert. Die nachstehende Formel zeigt dies.

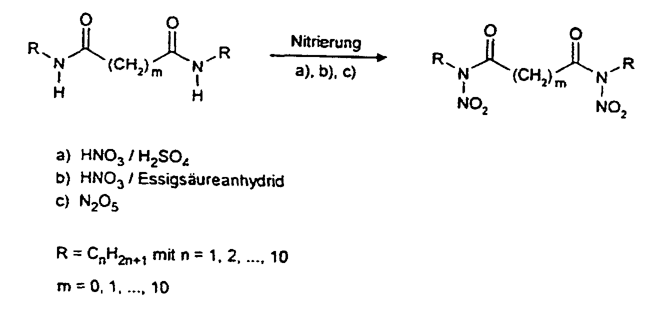

Geht man im zweiten Schritt von Diamiden mit zyklischen oder aromatischen Resten aus, erhält man Dinitroamid-Verbindungen mit den korrespondierenden Resten. Die Nitrierung erfolgt mit Hilfe üblicher Nitrierungsmittel, vorzugsweise mit Hilfe von Mischsäuren, Salpersäure, Essigsäureanhydrid oder Distickstoffpentoxyd, mit oder ohne Lösungsmittel. Die Temperatur sollte während der Zugabe der Nitrierungsmittel im Bereich von -20 bis +20°C liegen. Bei den flüssigen Dinitroverbindungen bilden sich zwei Phasen, die festen Dinitroverbindungen können abfiltriert werden.

In einem weiteren, dritten Schritt setzt man die Dialkyldinitroamide mit Methyl- und/oder Ethylamin in Wasser um. Hierbei entstehen als Nebenprodukt Dimethyl- und/oder Diethyldiamide, die nach der Nitrierung wiederum zur Darstellung von Methylund Ethylnitramin eingesetzt werden können, sowie nach Ansäuerung Alkylnitramine, deren Alkylrest denen des Dinitroamids entspricht. Den dritten Schritt zeigt die folgende Folge:

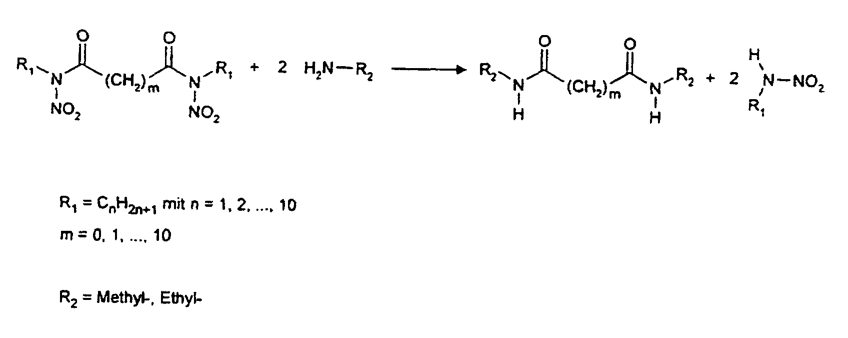

Die Isolierung der Alkylnitramine erfolgt vorzugsweise durch Extraktion mit Hilfe eines organischen Lösungsmittels, wie z.B. Diethylether, Dichlormethan, Methyltert-Buthylether (MTBE), Ethylacetat oder Toluol, wobei Ether bevorzugt wird.

Im vierten Schritt werden die isolierten Alkylnitramine in an sich bekannter Weise zu den Dinitrodiazaalkanen kondensiert. Geeignete Vorgehensweisen hierfür sind in der US-PS 4,476,322 und bei Tartakofsky et al a.a.O. offenbart. Ein bevorzugter Weg der Kondensierung ergibt sich aus Anspruch 10, wobei eine 50-98°ige Schwefelsäure eingesetzt wird. Das hierbei verwendte Lösungsmittel kann das gleiche sein, das schon im dritten Schritt benutzt wurde und ohne Entferung am Ende desselben weiter benutzt wird. Schematisch gibt den vierten Schritt die folgende Formel wieder:

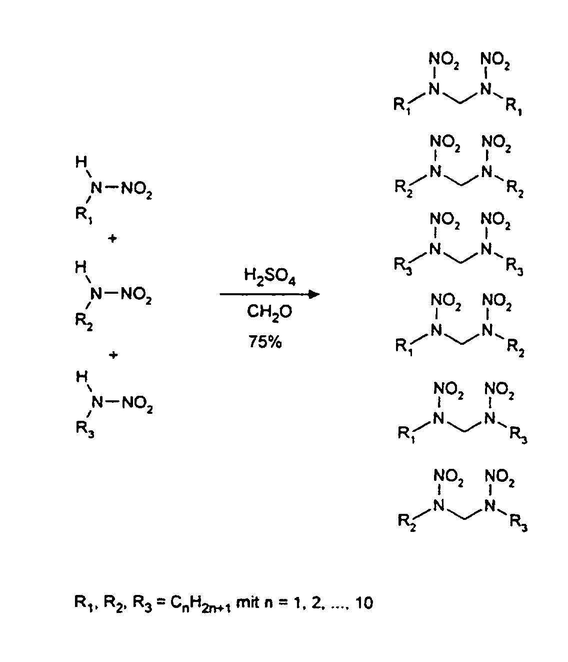

Die Erfindung zielt insbesondere auf die Herstellung eines eingangs schon angesprochenen Gemisches aus den drei Verbindungen 2,4-Dinitro-2,4-Diazapentan, 2,4-Dinitro-2,4-Diazahexan und 3,5-Dinitro-3,5-Diazaheptan, hier DNDA 57 genannt, weil dieses Gemisch für die Herstellung von Treibladungspulvern mit ausgeglichem Temperaturverhalten als besonders geeignet erscheint. Die gewünschte Zusammensetzung des Gemisches kann man bei der Kondensation durch die relativen Mengen der unterschiedlichen eingesetzten Nitramine steuern.

In diesem Zusammenhang ist die Weiterbildung der Erfindung nach Anspruch 12 besonders vorteilhaft. Hierbei werden Methylnitramin und Ethylnitramin gemeinsam im gleichen Verfahren hergestellt und ihr Verhältnis kann dabei von vorneherein entsprechend der gewünschten Zusammensetzung des DNDA 57 eingestellt werden, so daß die beiden Nitramine sofort ohne weitere Aufbereitung im vierten Syntheseschritt zu DNDA 57 kondensiert werden können.

Das erfindungsgemäße Verfahren hat gegenüber den eingangs behandelten Synthesewegen verschiedene Vorteile: Die Kosten für die Ausgangsmaterialien sind gering und die Ausgangsmaterialien stehen in großer Menge zur Verfügung. Die Ausbeuten sind relativ hoch. Man erhält isolierbare Zwischenstufen. Das Verfahren ist vergleichsweise einfach im technischen Bereich realisierbar. Schließlich kann es umweltverträglich durchgeführt werden, da ein Großteil der Reaktionen im wässrigen Medium erfolgt und sämtliche Abfallprodukte biologisch gut abbaubar sind.

Zur Erfindung gehoven ferner Dialkyldinitroamide einer höheren Dicarbonsäure, außerdem Dinitroamide einer Dicarbonsäure, bei welcher der Alkyl-Rest durch einen zyklischen oder aromatischen Rest ersetzt ist. Solche Stoffe werden als Zwischenprodukt bei der Durchführung des erfindungsgemäßen Verfahrens am Ende des zweiten Schrittes, also durch Nitrierung erhalten, wobei der Rest durch entsprechende Wahl des im ersten Schritt eingesetzten Amins gesteuert wird. Eine bevorzugte Verwendung dieser Stoffe, der Dialkyldinitroamide, liegt in der Verwendung als Zwischenprodukt für die Herstellung von Alkylnitraminen oder Dinitrodiazaalkanen, z.B. mit Hilfe der Schritte 3 oder der Schritte 3 und 4 des erfindungsgemäßen Verfahrens. Aus Chemical Abstract, Zitat 46: 904 G ist das Dimethyldinitroamid der Oxalsäure bereits bekannt, jedoch ohne Hinweis auf die obige Verwendbarkeit.

Im folgenden wird die Erfindung mit weiteren Einzelheiten näher erläutert, und zwar anhand von Beispielen für die Synthese von Methyl- und Ethylnitramin und für die Darstellung des daraus resultierenden energetischen Weichmachergemisches DNDA 57.

### 1) Synthese von N,N'-Dimethyloxalsäurediamid aus Oxalsäurediethylester und Methylamin

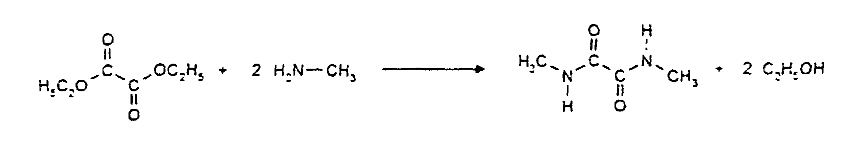

Zu 292 g (2.0 mol, d = 1.08, 270 ml) Oxalsäurediethylester werden unter Rühren 389 g (5.0 mol, d = 0.90, 432 ml) 40%ige Methylaminlösung zugetropft. Die Temperatur sollte dabei 80°C nicht übersteigen. Nach einer Stunde Nachreaktionszeit wird der farblose Feststoff abfiltriert, mit wenig Wasser gewaschen und getrocknet. Ausbeute: 125 g (1.1 mol, 54 %).

### 2) Synthese von N,N'-Diethyloxalsäurediamid aus Oxalsäurediethylester und Ethylamin

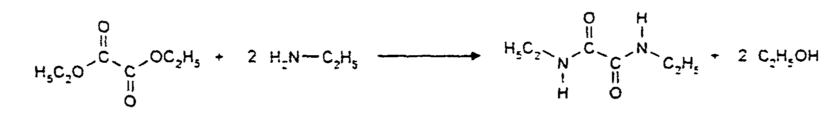

Zu 292 g (2.0 mol, d = 1.08) Oxalsäurediethylester werden unter Rühren 387 g (5.0 mol, d = 0.81) 70%ige Ethylaminlösung dazugetropft. Nach dem Zutropfen wird noch eine Stunde bei Raumtemperatur gerührt. Der farblose Feststoff wird abfiltriert, mit wenig Wasser gewaschen und getrocknet. Ausbeute: 154 g ( 1.1 mol, 53 %).

### 3) Synthese von Oxalsäure-bis-[methyl-nitro-amid]:

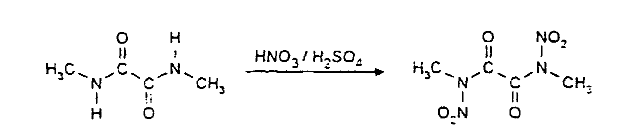

10.0 g (0.09 mol) Oxalsäure-bis-[methylamid] werden in 25 ml 96%iger HNO₃ gelöst und unter Vermeidung zu starker Erwärmung (25-45°C) mit 50 ml H₂SO₄ versetzt

Der entstehende Brei wird auf Eis gegossen, filtriert, mit Wasser neutral gewaschen und getrocknet Ausbeute 14.8 g (0.07 mol, 79 %), Schmp. 124°C aus Ethanol.

### 4) Synthese von Oxalsaure-bis-[ethyl-nitro-amid]:

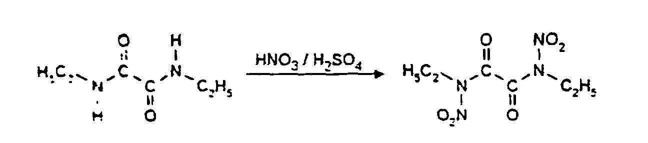

26.0 g (0.18 mol) Oxalsaure-bis-[ethylamid] werden in 50 ml 96%iger HNO₃ gelost und unter Vermeidung zu starker Erwärmung (25-45°C) mit 100 ml H₂SO₄ versetzt. Es bilden sich zwei Phasen. Die organische Phase wird abgetrennt, mit Wasser und gesättigter Natriumcarbonatlösung gewaschen. Ausbeute: 30.6 g (0.13 mol, 89 %).

### 5) Synthese von Methylnitramin:

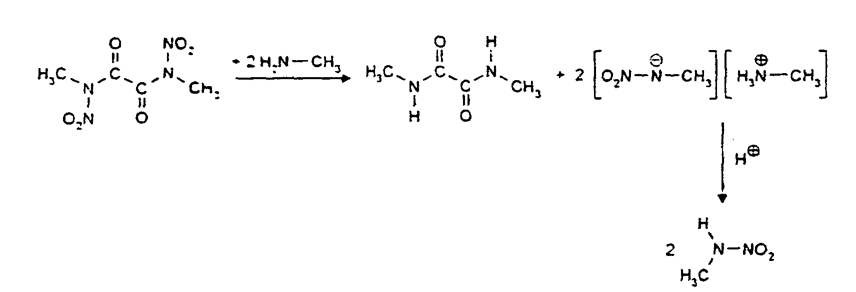

10.0 g (0 05 mol) Oxalsaure-bis-[methyl-nitro-amid] werden in kleinen Portionen mit 17.5 ml (0.23 mol) 40%iger Methylamin-Losung versetzt. Die Mischung erwärmt und verändert sich allmählich Nach ca. einer Stunde filtriert man vom zurückgebildeten Oxalsäure-bis-[methylamid] ab und wäscht mit wenig Wasser. Die wässrige Phase wird mit H₂SO₄ angesäuert, dabei entsteht Methylnitramin und Methylaminsulfat. Es wird 3mal mit je 50 ml Ether extrahiert. Nach dem Trocknen über MgSO₄ wird der Ether entfernt. Ausbeute: 6.2 g (0.08 mol, 82%)

### 6) Synthese von Ethylnitramin:

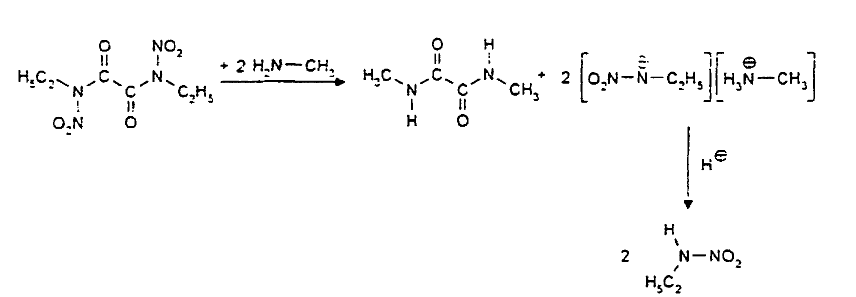

11.7 g (0.05 mol) Oxalsäure-bis-[ethyl-nitro-amid] werden in kleinen Portionen mit 17.5 ml (0.23 mol) 40%iger Methylamin-Lösung versetzt. Die Mischung erwärmt und verändert sich allmählich. Nach ca. einer Stunde filtriert man vom zurückgebildeten Oxalsäure-bis-[methylamid] ab und wäscht mit wenig Wasser. Die wässrige Phase wird mit H₂SO₄ angesäuert, dabei entsteht Ethylnitramin und Methylaminsulfat. Es wird 3mal mit je 50 ml Ether extrahiert. Nach dem Trocknen über MgSO₄ wird der Ether entfernt. Ausbeute: 8.9 g (0.10 mol, 99 %).

### 7) Umsetzung eines Gemisches aus Methyl- und Ethylnitramin zu DNDA 57

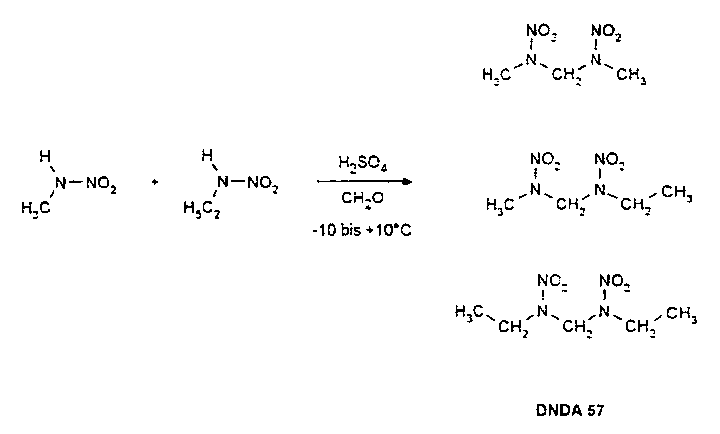

Zur Synthese von DNDA 57 werden 2.3 g Paraformaldehyd in 40 ml 75%iger Schwefelsäure vorgelegt und auf 0°C gekühlt. Eine Mischung von 7.2 g (95 mmol) Methylnitramin und 4.5 g (50 mmol) Ethylnitramin werden so zugetropft, daß die Temperatur der Reaktionslösung nicht über 5°C steigt. Nach einer Stunde Nachreaktionszeit wird auf Eiswasser gegossen und die wässrige Phase mit insgesamt ca. 50 ml Dichlormethan extrahiert. Die vereinigten organischen Phasen werden mit gesättigter Natriumcarbonatlösung gewaschen und über Magnesiumsulfat getrocknet. Nach dem Entfernen des Lösungsmittels erhält man DNDA 57 in einer Ausbeute von 10.3 g (83 %). Das Verhältnis der drei Komponenten stellt sich dabei wie folgt dar:

| | |
|---|---|
| 2,4-Dinitro-2,4-diazapentan | ca 45 % |
| 2,4-Dinitro-2,4-diazahexan | ca. 44 % |
| 3,5-Dinitro-3,5-diazaheptan | ca. 11 % |

### Übersicht DNDA-Synthese

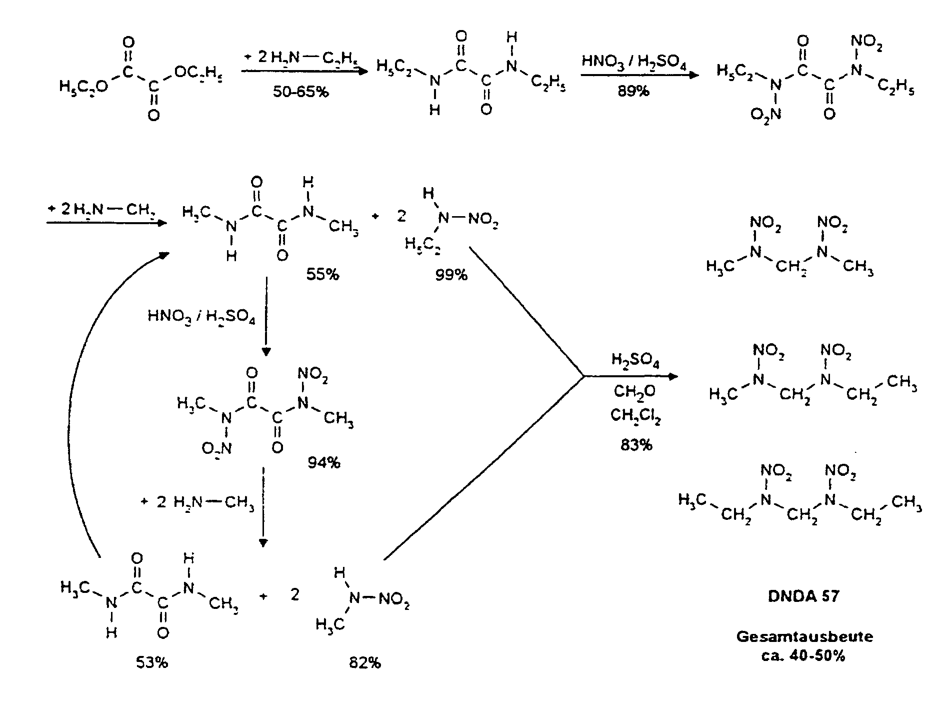

Die vorstehende Übersicht DNDA-Synthese zeigt, wie sich die einzelnen zuvor beschriebenen Schritte zu einem geschlossenen Verfahren für die Herstellung von DNDA 57 zusammenfügen. Ausgehend von Oxalsäure-Diethylester und Ethylamin wird zunächst Diethyloxalsäurediamid synthetisiert und hieraus durch Nitrierung mit Hilfe von Salpeter- und Schwefelsäure Oxalsäure-Diethyldinitroamid gewonnen. Letzteres wird mit Methylamin zu Dimethyloxalsäurediamid als Nebenprodukt und zu Ethylnitramin umgesetzt. Das Nebenprodukt wird wie zuvor nitriert, wodurch man Oxalsäure-Dimethyldinitroamid erhält. Durch Reaktion dieses Zwischenproduktes mit Methylamin erhält man wiederum - als Nebenprodukt - Dimethyloxalsäurediamid, das erneut der Nitrierung zugeführt wird, sowie Methlynitramin. Die beiden erhaltenen Nitramine werden gemeinsam durch Kondensation mit Hilfe von Schwefelsäure und Para-Formaldehyd in das gewünschte Gemisch DNDA 57 von drei Dinitrodiazaalkanen überführt. In der Übersicht sind die in den einzelnen Schritten erzielten Ausbeuten angegeben. Die Gesamtausbeute beträgt ca. 40-50%.

Eine Variante zu den oben dargestellten Synthesen 3) bis 6) ist die gleichzeitige Synthese von Methyl- und Enthylnitramin aus den beiden Dialkyloxalsäurediamiden

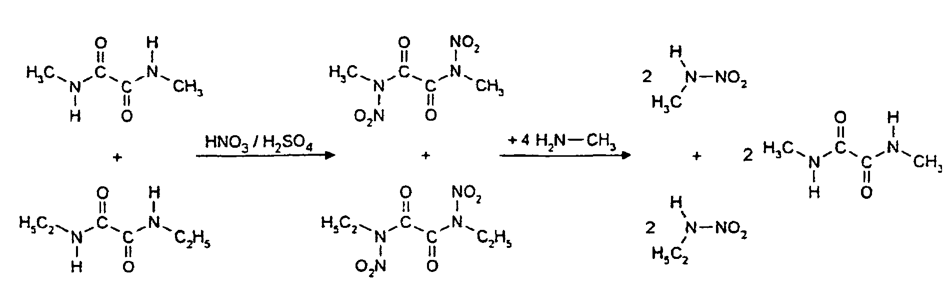

15.2 g (0.13 mol) Oxalsäure-bis-[methylamid] und 6.5 g (0.04 mol) Oxalsäure-bis-[ethylamid] werden in 50 ml 96%iger HNO₃ gelöst und unter Vermeidung zu starker Erwärmung (25-45°C) mit 100 ml H₂SO₄ versetzt. Der entstehende Brei wird auf Eis gegossen. filtriert und mit Wasser neutral gewaschen. Die entstandenen Oxalsäurediamide werden in kleinen Portionen mit insgesamt ca. 60 ml (0.79 mol) 40%iger Methylamin-Lösung versetzt. Die Mischung erwärmt und verändert sich allmählich. Nach ca. einer Stunde filtriert man vom zurückgebildeten Oxalsäure-bis-[methylamid] ab und wäscht mit wenig Wasser. Die wässrige Phase wird mit H₂SO₄ angesäuert, dabei entsteht Methylnitramin, Ethylnitramin und Methylaminsulfat. Es wird 3mal mit je 50 ml Ether extrahiert. Nach dem Trocknen über MgSO₄ wird der Ether entfernt. Die beiden Nitramine werden im gewünschten Verhältnis in einer Ausbeute von 61 % erhalten und können sofort ohne weitere Aufbereitung im nächsten Syntheseschritt eingesetzt werden.

## Patentansprüche

1. Verfahren zur Herstellung von Dinitrodiazaalkanen aus Alkylaminen und Estern,
**gekennzeichnet durch** die Kombination folgender Schritte:
1. ein Diester einer Dicarbonsäure wird mit einem Alkylamin im wässrigen Milieu in das korrespondierende Dialkyldiamid der Dicarbonsäure umgesetzt,
2. das erhaltene Dialkyldiamid wird mittels üblicher Nitrirungsmittel zu dem korrespondierenden Dialkyldinitroamid der Dicarbonsäure nitriert,
3. das erhaltene Dialkyldinitroamid wird in das korrespondierende Alkylnitramin umgesetzt, indem das Dialkyldinitroamid im wässrigen Milieu mit Methyl- und/oder Ethylamin versetzt, das dabei entstandene Dimethyl- und/oder Diethyldiamid der Dicarbonsäure abgetrennt, das verbleibende Produkt angesäuert und dann daraus das Alkylnitramin extrahiert wird,
4. das isolierte Alkylnitramin wird in an sich bekannter Weise zu den Dinitrodiazaalkanen kondensiert.

2. Verfahren nach Anspruch 1,
bei welchem man von einem Dialkylester einer Dicarbonsäure, vorzugsweise einer aliphatischen Dicarbonsäure, ausgeht.

3. Verfahren nach Anspruch 2,
bei welchem man von Oxalsäure-Diethylester ausgeht.

4. Verfahren nach Anspruch 1, 2 oder 3,
bei welchem man im ersten Schritt Methyl- und/oder Ethylamin, vorzugsweise Ethylamin, einsetzt.

5. Verfahren nach Anspruch 1, 2, 3 oder 4,
bei welchem man im dritten Schritt Methylamin einsetzt.

6. Verfahren nach einem der Ansprüche 2 bis 5, bei welchem man im ersten Schritt das Alkyl-Amin in wässriger Lösung dem Dialkylester bei einer Temperatur zwischen 0 und 80°C allmählich zugibt und das Reaktionsprodukt nach einer Nachreaktionszeit von 0,5 bis 3 Stunden, vorzugsweise 1 bis 2 Stunden, abfiltriert.

7. Verfahren nach einem der Ansprüche 1 bis 6,
bei welchem man im zweiten Schritt als Nitrierungsmittel Mischsäure, Salpetersäure, Essigsäureanhydrid oder Distickstoffpentoxid mit oder ohne Lösungsmittel verwendet.

8. Verfahren nach Anspruch 7,
bei welchem man das Dialkyldiamid in Salpetersäure löst und bei einer Temperatur unter 20°C mit konzentrierter Schwefelsäure versetzt, sodann das Reaktionsprodukt auf Eis gießt und danach filtriert oder abtrennt.

9. Verfahren nach einem der Ansprüche 1 bis 8,
bei welchem man im dritten Schritt das verbleibende Produkt mit konzentrierter Schwefelsäure ansäuert und danach das Alkylnitramin mit einem organischen Lösungsmittel, vorzugsweise mit Ether, extrahiert.

10. Verfahren nach einem der Ansprüche 1 bis 9,
bei welchen man im vierten Schritt Para-Formaldehyd in konzentrierter Schwefelsäure vorlegt, das Alkylnitramin bei einer Temperatur zwischen -20 und +20°C allmählich zugibt, dann mit Wasser verdünnt und mit einem organischen Lösungsmittel extrahiert und schließlich die organischen Phasen wäscht und das Lösungsmittel entfernt.

11. Verfahren nach einem der Ansprüche 1 bis 10,
bei welchem man das im dritten Schritt als Nebenprodukt abgetrennte Dimethyl- und/oder Diethyldiamid erneut im zweiten Schritt nitriert und im dritten Schritt zur Darstellung von Methyl- und/oder Ethylnitramin verwendet.

12. Verfahren nach einem der Ansprüche 4 bis 11,
bei welchem man im zweiten Schritt Dimethyldiamid und Diethyldiamid gemeinsam nitriert und die beiden Reaktionsprodukte im dritten Schritt gemeinsam in Methlynitramin und Ethylnitramin umsetzt.

13. Verfahren zur Herstellung von Alkylnitraminen aus Alkylaminen und Estern,
**gekennzeichnet durch**
die Schritte 1 bis 3 gemäß Anspruch 1.

## Claims

1. A method of synthesis of dinitro-diaza-alkanes from alkylamines and esters,
**characterized by** a combination of the following steps:
1. reacting a diester of a dicarboxylic acid with an alkylamine in an aqueous medium to yield the corresponding dialkyldiamide of the dicarboxylic acid;
2. nitrating the resulting dialkyldiamide by means of the usual nitration agents to form the corresponding dialkyldinitroamide of dicarboxylic acid;
3. reacting the resulting dialkyldinitroamide to form the corresponding alkylnitroamine by mixing the dialkyldinitroamide in an aqueous medium with methylamine and/or ethylamine, separating the resulting dimethyldiamide and/or diethyldiamide of the dicarboxylic acid, acidifying the remaining product and then extracting the alkylnitroamine from that;
4. condensing the isolated alkylnitroamine to form the dinitro-diaza-alkanes in the known manner.

2. A method according to Claim 1,
whereby the process starts with a dialkyl ester of a dicarboxylic acid, preferably an aliphatic dicarboxylic acid.

3. A method according to Claim 2,
whereby the process starts with oxalic acid diethyl ester.

4. A method according to Claims 1, 2 or 3,
whereby methylamine and/or ethylamine, preferably ethylamine, is used in the first step.

5. A method according to Claims 1, 2, 3 or 4,
whereby methylamine is used in the third step.

6. A method according to one of Claims 2 through 5,
whereby in the first step, the alkylamine in an aqueous solution is added gradually to the dialkyl ester at a temperature between 0 °C and 80 °C, and the reaction product is filtered out after a secondary reaction time of 0.5 to 3 hours, preferably one to two hours.

7. A method according to one of Claims 1 through 6,
whereby in the second step lactic acid, nitric acid, acetic anhydride or dinitrogen pentoxide with or without a solvent is used as the nitration agent.

8. A method according to Claim 7,
whereby the dialkyldiamide is dissolved in nitric acid, and mixed with concentrated sulfuric acid at a temperature below 20 °C, then the reaction product is poured onto ice and then filtered or separated.

9. A method according to one of Claims 1 through 8,
whereby in the third step the remaining product is acidified with concentrated sulfuric acid, and then the alkylnitroamine is extracted with an organic solvent preferably with ether.

10. A method according to one of Claims 1 through 9,
whereby the fourth step begins with paraformaldehyde in the concentrated sulfuric acid to which alkylnitroamine is added gradually at a temperature between -20 °C and +20 °C, then diluting with water and extracting with an organic solvent and finally washing the organic phases and removing the solvent.

11. A method according to one of Claims 1 through 10,
whereby the dimethyldiamide and/or diethyldiamide which is separated as a by-product in the third step is nitrated again in the second step and used in the third step to synthesize methylnitroamine and ethylnitroamine.

12. A method according to one of Claims 4 through 11,
whereby in the second step dimethyldiamide and diethyldiamide are nitrated together, and the two reaction products are jointly reacted to form methylnitroamine and ethylnitroamine in the third step.

13. A method of synthesis of alkylnitroamines from alkylamines and esters,
**characterized by** steps 1 through 3 according to Claim 1.

## Revendications

1. Procédé de préparation de dinitrodiazaalcanes à partir d'alcoylamines et d'esters,
**caractérisé par** la combinaison des stades suivants :
1.On transforme un diester d'un acide dicarboxylique par une alcoylamine en milieu aqueux en le dialcoyldiamide correspondant de l'acide dicarboxylique.
2. On nitre le dialcoyldiamide obtenu au moyen d'un agent nitrant habituel en le dialcoyldinitroamide correspond de l'acide dicarboxylique.
3. On transforme le dialcoyldinitroamide obtenu en l'alcoylnitramine correspondante en mettant le dialcoyldinitroamide à réagir en milieu aqueux sur la méthylamine et/ou sur l'éthylamine, on sépare le diméthyldiamide et/ou le diéthyldiamide de l'acide dicarboxylique qui est obtenu ; on acidule le produit restant, puis on en extrait l'alcoylnitramine.
4. On condense d'une manière en soi connue l'alcoylnitramine isolée en les dinitrodiazaalcanes

2. Procédé suivant la revendication 1,
dans lequel on part d'un ester dialcoylique d'un acide dicarboxylique, de préférence d'un acide dicarboxylique aliphatique.

3. Procédé suivant la revendication 2,
dans lequel on part de l'ester diéthylique de l'acide oxalique.

4. Procédé suivant la revendication 1, 2 ou 3,
dans lequel on utilise au premier stade de la méthylamine e/ou de l'éthylamine, de préférence de l'éthylamine

5. Procédé suivant la revendication 1, 2, 3 ou 4,
dans lequel on utilise au troisième stade de la méthylamine.

6. Procédé suivant l'une des revendications 2 à 5,
dans lequel on ajoute peu à peu, à une température comprise entre 0 et 80° C, au premier stade, l'alcoylamine en solution aqueuse à l'ester dialcoylique et l'on filtre le produit de réaction après une durée de réaction d'une demi-heure à 3 heures, de préférence de 1 à 2 heures.

7. Procédé suivant l'une des revendications 1 à 6,
dans lequel on utilise au deuxième stade comme agent nitrant le mélange sulfonitrique, l'acide nitrique, l'anhydride acétique ou le pentoxide d'azote.

8. Procédé suivant la revendication 7,
dans lequel on dissout le dialcoyldiamide dans l'acide nitrique et on le mélange à une température inférieure à 20° C à de l'acide sulfurique concentré, on verse immédiatement le produit de réaction sur de la glace, puis on le filtre ou on le sépare.

9. Procédé suivant l'une des revendications 1 à 8,
dans lequel au troisième stade on acidule le produit restant par de l'acide sulfurique concentré et ensuite on extrait l'alcoylnitramine par un solvant organique, de préférence par de l'éther.

10. Procédé suivant l'une des revendications 1 à 9,
dans lequel on met, au quatrième stade, du paraformaldéhyde dans de l'acide sulfurique concentré, on ajoute peu à peu l'alcoylnitramine à une température comprise entre -20° et + 20° C, puis on dilue par de l'eau et on fait subir une extraction par un solvant organique et enfin on lave les phases organiques et on élimine le solvant.

11. Procédé suivant l'une des revendications 1 à 10,
dans lequel on nitre, à nouveau, au deuxième stade, le diméthyldiamide et/ou le diéthyldiamide séparé en tant que sous-produit au troisième stade et on l'utilise au troisième stade pour la préparation de méthylnitramine et/ou d'éthylnitramine.

12. Procédé suivant l'une des revendications 4 à 11,
dans lequel on nitre conjointement au deuxième stade du diméthyldiamide et du diéthyldiamide et l'on transforme les deux produits de réaction au troisième stade conjointement en méthylnitramine et en en éthylnitramine.

13. Procédé de préparation d'alcoylnitramines à partir d'alcoylamines et d'esters,
**caractérisé par** les stades 1 à 3 suivant la revendication 1.
